# EUROPEAN PATENT APPLICATION

(11) **EP 4 011 450 A1**
(43) Date of publication of application: **15.06.2022**
(21) Application number: 20850966.1
(22) Date of filing: 06.08.2020
(51) Int. Cl.: A61P 27/02, A61P 27/04, A61K 31/519

(54) **OPHTHALMIC COMPOSITION FOR PROMOTING TEAR SECRETION**

(30) Priority: 07.08.2019 JP 2019145670; 27.12.2019 JP 2019239605
(71) Applicant: Rohto Pharmaceutical Co., Ltd., Osaka-shi, Osaka 544-8666 (JP)
(72) Inventor: TAKAI Yoshihiro, Osaka-shi, Osaka 544-8666 (JP); KUROSE Takahiro, Osaka-shi, Osaka 544-8666 (JP)
(74) Representative: Mewburn Ellis LLP
(86) International application number: PCT/JP2020/030231
(87) International publication number: WO 2021/025129

(57) **Abstract**

The present invention relates to an ophthalmic composition for promoting tear secretion, the composition comprising 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof.

## Description

### Technical Field

The present invention relates to an ophthalmic composition for promoting tear secretion.

### Background Art

A keratoconjunctival epithelial disorder is caused by endogenous diseases such as dry eye or exogenous diseases due to wear of contact lenses or the like, and is a disease exhibiting symptoms such as lacrimation, foreign body sensation, eye pain, and bloodshot eyes. As a therapeutic agent for the keratoconjunctival epithelial disorders, eye drops containing sodium hyaluronate having the action of promoting corneal epithelial extension and retaining moisture have been placed on the market. It has been reported that diquafosol sodium, a therapeutic agent for dry eye, promoted secretion of tear and ameliorated corneal epithelial disorders in a dry eye model of rats (e.g., Non Patent Literature 1).

On the other hand, Janus kinase (JAK) is a non-receptor tyrosine kinase playing an important role in intracellular immune activation signaling, and it is expected that drugs having Janus kinase inhibitory activity will ameliorate autoimmune diseases and allergic diseases by suppressing excessive activation of immune responses. Here, as one of compounds having Janus kinase inhibitory action, 3-[(3S,4R)-3-methyl-6-(7H-pyrrolo[2,3-d]pyrimidin-4-yl)-1,6-diazaspir o[3.4]octan-1-yl]-3-oxopropanenitrile (non-proprietary name: delgocitinib) is known (e.g., Patent Literature 1).

### Citation List

### Patent Literature

Patent Literature 1: International Publication No. WO 2017/006968

### Non Patent Literature

Non Patent Literature 1: Package Insert of DIQUAS Ophthalmic Solution 3% (Revised: November 2017)

### Summary of Invention

### Technical Problem

An object of the present invention is to provide a novel ophthalmic composition that can promote secretion of tear.

### Solution to Problem

As a result of diligent study in order to solve the above problem, the present inventors have found that 3-[(3S,4R)-3-methyl-6-(7H-pyrrolo[2,3-d]pyrimidin-4-yl)-1,6-diazaspir o[3.4]octan-1-yl]-3-oxopropanenitrile unexpectedly promotes secretion of tear and ameliorate a keratoconjunctival epithelial disorder. The present invention is based on this finding and provides each of the following inventions.
[1] An ophthalmic composition for promoting tear secretion, the composition comprising 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof.
[2] The ophthalmic composition according to [1], wherein the composition is used to ameliorate a keratoconjunctival epithelial disorder.
[3] The ophthalmic composition according to [1], wherein the composition is used to ameliorate dry eye.
[4] An ophthalmic composition for ameliorating a keratoconjunctival epithelial disorder, the composition comprising 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof.
[5] The ophthalmic composition according to [2] or [4], wherein the keratoconjunctival epithelial disorder is caused by dry eye.
[6] The ophthalmic composition according to any one of [1] to [5], wherein a content of 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof is 0.01% by mass to 1% by mass based on a total amount of the ophthalmic composition.
[7] The ophthalmic composition according to any of [1] to [6], wherein the composition is an eye drop.
[8] A method for promoting secretion of tear, comprising administering to a subject 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof, or an ophthalmic composition comprising the same.
[9] A method for ameliorating a keratoconjunctival epithelial disorder, comprising administering to a subject 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof, or an ophthalmic composition comprising the same.
[10] The method according to [9], wherein the keratoconjunctival epithelial disorder is caused by dry eye.
[11] Use of 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof, for the production of an ophthalmic composition for promoting tear secretion.
[12] Use of 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof, for the production of an ophthalmic composition for ameliorating a keratoconjunctival epithelial disorder.
[13] The use according to [12], wherein the keratoconjunctival epithelial disorder is caused by dry eye.
[14] 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof, for use in promoting tear secretion.
[15] 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof, for use in ameliorating a keratoconjunctival epithelial disorder.
[16] The compound or salt thereof according to [15], wherein the keratoconjunctival epithelial disorder is caused by dry eye.

### Advantageous Effects of Invention

According to the present invention, it is possible to provide an ophthalmic composition that can promote secretion of tear.

### Brief Description of Drawings

[Figure 1] Figure 1 is a graph illustrating tear secretion promoting action by single ophthalmic administration of delgocitinib using normal rabbits in Test Example 1.
[Figure 2] Figure 2 is a graph illustrating tear secretion promoting action by single ophthalmic administration of delgocitinib using normal mice in Test Example 2.
[Figure 3] Figure 3 (A) is a graph illustrating tear secretion promoting action by single ophthalmic administration of delgocitinib using normal rats in Test Example 3. Figure 3 (B) is a graph illustrating tear secretion promoting action by single ophthalmic administration of delgocitinib using lacrimal gland excised rats in Test Example 3.
[Figure 4] Figure 4 is a graph illustrating tear volume increasing action by repeated ophthalmic administration of delgocitinib using lacrimal gland excised rats in Test Example 4.
[Figure 5] Figure 5 is a graph illustrating the action of ameliorating keratoconjunctival epithelial disorders by repeated ophthalmic administration of delgocitinib using lacrimal gland excised rats in Test Example 5.

### Description of Embodiments

Hereinafter, embodiments of the present invention will be explained in detail. However, the present invention is not limited to the following embodiments.

The ophthalmic composition of the present embodiment comprises 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof.

Here, 3-[(3S,4R)-3-methyl-6-(7H-pyrrolo[2,3-d]pyrimidin-4-yl)-1,6-diazaspir o[3.4]octan-1-yl]-3-oxopropanenitrile (non-proprietary name: delgocitinib) is a compound represented by the following formula: 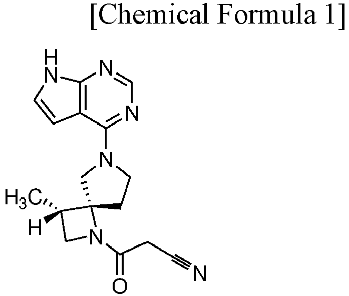 (hereinafter, this compound is also referred to as "delgocitinib"). Delgocitinib or a salt thereof can be produced by methods described in, for example, International Publication No. WO 2017/006968 and International Publication No. WO 2018/117151.

The salt of delgocitinib is not particularly limited as long as it is medicinally, pharmacologically (pharmaceutically) or physiologically acceptable. Specific examples of such salts include salts with inorganic acids, salts with organic acids, salts with inorganic bases, salts with organic bases, salts with acidic amino acids, and salts with basic amino acids.

Examples of the salts with inorganic acids include salts with hydrochloric acid, hydrobromic acid, sulfuric acid, nitric acid, and phosphoric acid. Examples of the salts with organic acids include salts with acetic acid, succinic acid, fumaric acid, maleic acid, tartaric acid, citric acid, lactic acid, stearic acid, benzoic acid, methanesulfonic acid (mesylic acid), ethanesulfonic acid, and p-toluenesulfonic acid. Examples of the salts with inorganic bases include alkali-metal salts such as sodium and potassium salts; alkaline-earth metal salts such as calcium and magnesium salts; aluminum salts; and ammonium salts. Examples of the salts with organic bases include salts with diethylamine, diethanolamine, meglumine, and N,N-dibenzylethylenediamine. Examples of the salts with acidic amino acids include salts with aspartic acid and glutamic acid. Examples of the salts with basic amino acids include salts with arginine, lysine, and ornithine.

Delgocitinib or a salt thereof exerts an effect of promoting the secretion of tear. Accordingly, as an embodiment of the present invention, an ophthalmic composition for promoting tear secretion comprising delgocitinib or a salt thereof is provided. In addition, it is considered that promotion of tear secretion is a major factor in the amelioration of keratoconjunctival epithelial disorders. Accordingly, as an embodiment of the present invention, an ophthalmic composition for ameliorating keratoconjunctival epithelial disorders comprising delgocitinib or a salt thereof is provided. It has never been reported that the secretion of tear is promoted by inhibition of Janus kinase.

The ophthalmic composition of the present embodiment can be used for any of keratoconjunctival epithelial disorders caused by endogenous diseases such as dry eye (dry eye syndrome), Sjogren's syndrome, and Stevens-Johnson syndrome, or keratoconjunctival epithelial disorders caused by exogenous diseases such as post-operative diseases and diseases caused by medication, trauma, wear of contact lenses, or the like. Among them, it is preferable to use the ophthalmic composition of the present embodiment for the amelioration of keratoconjunctival epithelial disorders caused by endogenous factors and more preferable to use it for the amelioration of keratoconjunctival epithelial disorders caused by dry eye because the ophthalmic composition promotes the secretion of tear by containing delgocitinib or a salt thereof. The dry eye that causes the keratoconjunctival epithelial disorders may be caused by autoimmune diseases such as Sjogren's syndrome or may be caused by factors other than the autoimmune diseases.

The ophthalmic composition of the present embodiment can also be used to ameliorate dry eye because it promotes the secretion of tear by containing delgocitinib or a salt thereof. The dry eye may be caused by autoimmune diseases such as Sjogren's syndrome or may be caused by factors other than the autoimmune diseases.

The content of delgocitinib or a salt thereof in the ophthalmic composition of the present embodiment is not particularly limited, and is set as appropriate in accordance with the type and content of other ingredients, formulation forms, and the like. In terms of the content of ingredient (A), from viewpoint of exerting effects by the present invention more remarkably, for example, the total content of delgocitinib or a salt thereof may be 0.001% by mass to 10% by mass, 0.001% by mass to 5% by mass, 0.003% by mass to 3% by mass, 0.005% by mass to 1% by mass, 0.01% by mass to 0.5% by mass, 0.015% by mass to 0.4% by mass, 0.02% by mass to 0.3% by mass, or 0.03% by mass to 0.3% by mass, based on the total amount of the ophthalmic composition of the present embodiment.

In the ophthalmic composition of the present embodiment, various additives may be selected as appropriate according to the usual method and added in an appropriate amount in combination with one or more of them, depending on the formulation form within the range that does not impair the effects of the present invention.

The pH of the ophthalmic composition of the present embodiment is not particularly limited, as long as it is within a medicinally, pharmacologically (pharmaceutically), or physiologically acceptable range.

The ophthalmic composition of the present embodiment can be prepared, for example, by adding and mixing delgocitinib or a salt thereof and, if necessary, other ingredients to obtain a desired content. Specifically, the composition can be prepared by dissolving or suspending the above ingredients in purified water and then sterilizing it by filtration sterilization, for example.

The ophthalmic composition of the present embodiment can take various dosage forms depending on the purpose, and examples thereof include solutions, gels, and semi-solids (ointment, etc.). Among them, solutions are preferable, and aqueous solutions are more preferable.

The ophthalmic composition of the present embodiment can be used, for example, as an eye drop (also referred to as an ophthalmic solution or an eye lotion; the eye drops includes artificial tears and eye drops that can be instilled while wearing contact lenses).

When the ophthalmic composition of the present embodiment is an eye drop, the dosage and administration are not particularly limited as long as they are effective and has fewer side effects.

As an embodiment of the present invention, a method for promoting secretion of tear comprising administering to a subject delgocitinib or a salt thereof, or an ophthalmic composition comprising the same is provided. As an embodiment of the present invention, a method for ameliorating a keratoconjunctival epithelial disorder comprising administering to a subject delgocitinib or a salt thereof, or an ophthalmic composition comprising the same is also provided.

As an embodiment of the present invention, the use of delgocitinib or a salt thereof for the production of an ophthalmic composition for promoting tear secretion is provided. As an embodiment of the present invention, the use of delgocitinib or a salt thereof for the production of an ophthalmic composition for ameliorating a keratoconjunctival epithelial disorder is also provided.

As an embodiment of the present invention, delgocitinib or a salt thereof for use in promoting tear secretion is provided. As an embodiment of the present invention, delgocitinib or a salt thereof for use in ameliorating a keratoconjunctival epithelial disorder is also provided.

### Examples

Hereinafter, the present invention will be explained specifically based on Test Examples, but the present invention is not limited to these examples. Note that in the following Test Examples, unless otherwise described, the content unit "%" means "w/v%" and is synonymous with "g/100 mL".

### [Test Example 1: Tear Secretion Promoting Action by Single Ophthalmic Administration of Delgocitinib Using Normal Rabbits]

The tear secretion promoting action of delgocitinib was evaluated using normal rabbits.

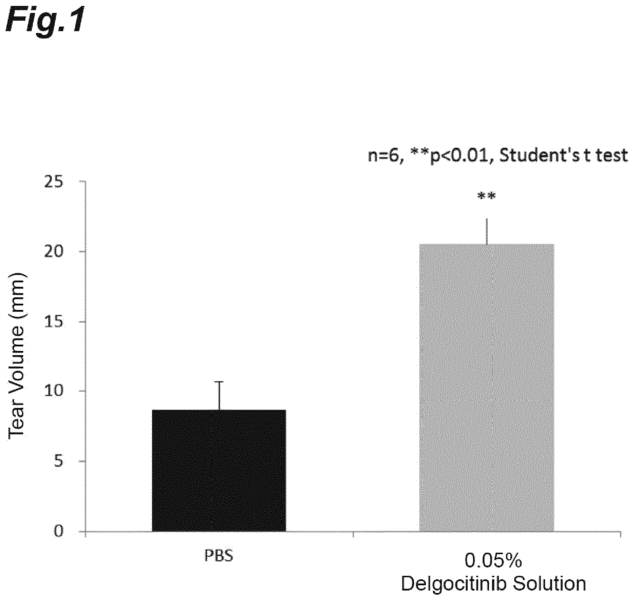

Delgocitinib was dissolved and stirred in PBS (Kohjin Bio Co., Ltd.) to prepare a 0.05% delgocitinib solution. Male Japanese white rabbits (Oriental Yeast Co., Ltd.) were placed in retention tubes, and the PBS or the 0.05% delgocitinib solution was instilled at a dose of 50 µL/eye. Fifteen minutes later, a Schirmer's test paper was inserted into conjunctival sac on the corner of the eye. One minute later, the test paper was pulled out, and the length of the wet part of the test paper was measured, which was used as a tear volume. The results are shown in Figure 1.

As shown in Figure 1, the tear volume was significantly increased in the delgocitinib solution group compared with the PBS group (Student's t test).

### [Test Example 2: Tear Secretion Promoting Action by Single Ophthalmic Administration of Delgocitinib Using Normal Mice]

The tear secretion promoting action of delgocitinib was evaluated using normal mice.

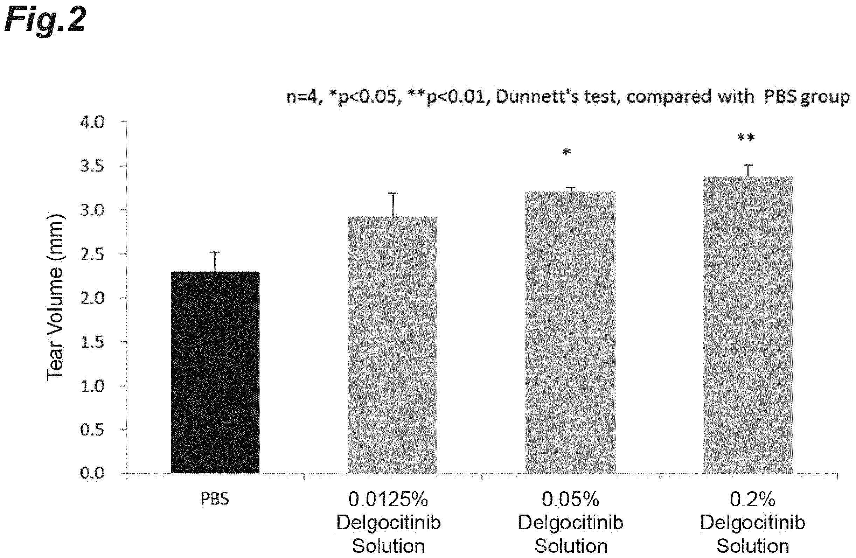

Delgocitinib was dissolved and stirred in PBS (Kohjin Bio Co., Ltd.) to prepare delgocitinib solutions at each concentration (0.0125%, 0.05%, and 0.2%). Female C57BL/6J mice (Japan SLC, Inc.) were retained, and the PBS or the delgocitinib solutions at each concentration were instilled at a dose of 3 µL/eye. Fifteen minutes later, a phenol red thread (ZONE-QUICK, AYUMI Pharmaceutical Corporation) was inserted into conjunctival sac on the corner of the eye. Fifteen seconds later, the phenol red thread was pulled out, the length of the wet part of the phenol red thread was measured, and this measured value was used as the tear volume. The results are shown in Figure 2.

As shown in Figure 2, the tear volume was increased in the delgocitinib solution group at each concentration compared with the PBS group, and a significant increase was observed especially in the 0.05% and 0.2% groups (Dunnett's test).

### [Test Example 3: Tear Secretion Promoting Action by Single Ophthalmic Administration of Delgocitinib Using Normal Rats and Lacrimal Gland Excised Rats]

The tear secretion promoting action of delgocitinib was evaluated using normal rats and lacrimal gland excision rats.

As a test substance, a 0.05% delgocitinib solution (delgocitinib as the active ingredient and sodium chloride, potassium chloride, sodium hydroxide, boric acid, and chlorhexidine gluconate as additives were combined, dissolved and stirred in purified water) was prepared. As a control substance, pilocarpine hydrochloride (Tokyo Chemical Industry Co., Ltd.) was dissolved and stirred in saline (OTSUKA NORMAL SALINE, Otsuka Pharmaceutical Factory, Inc.) to prepare a 3% pilocarpine solution.

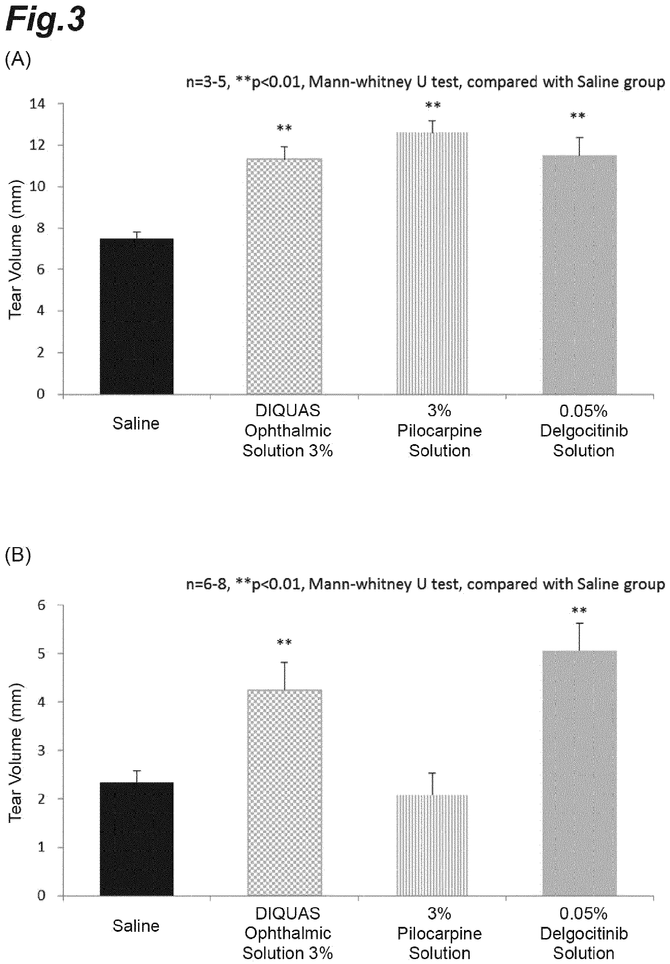

Into normal male Sprague-Dawley(SD) rats (Japan SLC, Inc.) and SD rats whose main and accessory lacrimal glands had been excised 1 week earlier, saline, the DIQUAS ophthalmic solution 3% (Santen Pharmaceutical Co., Ltd.), the 3% pilocarpine solution, or the 0.05% delgocitinib solution was instilled at a dose of 5 µL/eye. Ten minutes later, anesthesia was induced using somnopentyl (Kyoritsu Seiyaku Corporation), and another 5 minutes later, a Schirmer's test paper was inserted into conjunctival sac on the corner of the eye. One minute later, the test paper was pulled out, and the length of the wet part of the test paper was measured, and this measured value was used as a tear volume. The results are shown in Figure 3.

As shown in Figure 3, in the normal rats, the tear volume was significantly increased in all the DIQUAS ophthalmic solution 3% group, the 3% pilocarpine solution group, and the 0.05% delgocitinib solution group, compared with the saline group (Mann-whitney U test). On the other hand, in the lacrimal gland excised rats, the tear volume was significantly increased only in the DIQUAS ophthalmic solution 3% group and the 0.05% delgocitinib solution group, compared with the saline group (Mann-whitney U test).

### [Test Example 4: Tear Volume Increasing Action by Repeated Ophthalmic Administration of Delgocitinib Using Lacrimal Gland Excised Rats]

The tear secretion volume increasing action of delgocitinib was evaluated using lacrimal gland excised rats as a dry eye model.

Delgocitinib was dissolved and stirred in PBS (Kohjin Bio Co., Ltd.) to prepare delgocitinib solutions (0.0125%, 0.05%, and 0.2%) at each concentration.

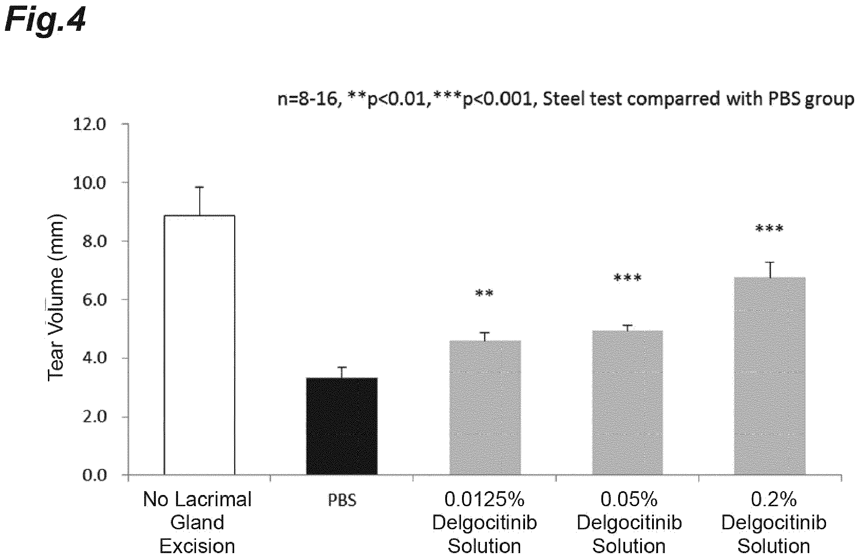

Into normal male Sprague-Dawley (SD) rats (Japan SLC, Inc.) and SD rats whose main lacrimal gland had been excised 1 week earlier, the PBS or the delgocitinib solutions at each concentration were repeatedly instilled four times a day at a dose of 5 µL/eye. Four weeks after the start of ophthalmic instillation, the PBS or the delgocitinib solutions at each concentration were instilled at a dose of 5 µL/eye, anesthesia was induced using somnopentyl (Kyoritsu Seiyaku Corporation) 10 minutes later, and another 5 minutes later, a Schirmer's test paper was inserted into conjunctival sac on the corner of the eye. One minute later, the test paper was pulled out, and the length of the wet part of the test paper was measured, and this measured value was used as a tear volume. The results are shown in Figure 4.

As shown in Figure 4, the tear volume was significantly increased in the delgocitinib solution group at each concentration compared with the PBS group. (Steel test).

### [Test Example 5: Action of Ameliorating Keratoconjunctival Epithelial Disorders by Repeated Ophthalmic Administration of Delgocitinib Using Lacrimal Gland Excised Rats]

The action of ameliorating keratoconjunctival epithelial disorders of delgocitinib was evaluated using rats with lacrimal gland excision used as a dry eye model.

Fluorescein sodium salt (Sigma-Aldrich Japan) was dissolved in OTSUKA NORMAL SALINE (Otsuka Pharmaceutical Factory, Inc.) to prepare a 10 mg/mL fluorescein staining solution.

Delgocitinib was dissolved and stirred in PBS (Kohjin Bio Co., Ltd.) to prepare delgocitinib solutions at each concentration (0.0125%, 0.05%, and 0.2%).

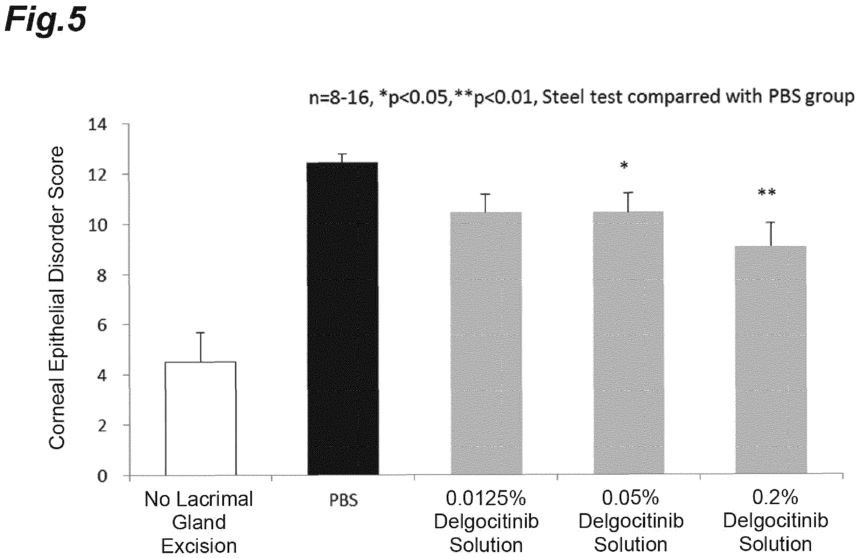

Into male SD rats (Japan SLC, Inc.) whose main lacrimal gland had been excised 1 week earlier, the PBS or the delgocitinib solutions at each concentration were repeatedly instilled four times a day at a dose of 5 µL/eye. Four weeks after the start of ophthalmic instillation, the 10 mg/mL fluorescein staining solution was instilled at a dose of 1 µL/eye. After induction of anesthesia with an ISOFLURANE inhalation solution (Pfizer Japan Inc.), a photograph was taken with a fluorescence microscope (Leica Microsystems). The cornea captured in the photograph was divided into four sections, the staining score of each section was scored on a scale of 0 to 5, and the total score of four sections was used as the corneal epithelial disorder score for the eye. The results are shown in Figure 5.

As shown in Figure 5, the corneal epithelial disorder score was significantly decreased in the delgocitinib solution groups at each concentration compared with the PBS group. (Steel test).

## Claims

1. An ophthalmic composition for promoting tear secretion, the composition comprising 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof.

2. The ophthalmic composition according to claim 1, wherein the composition is used to ameliorate a keratoconjunctival epithelial disorder.

3. The ophthalmic composition according to claim 1, wherein the composition is used to ameliorate dry eye.

4. An ophthalmic composition for ameliorating a keratoconjunctival epithelial disorder, the composition comprising 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof.

5. The ophthalmic composition according to claim 2 or 4, wherein the keratoconjunctival epithelial disorder is caused by dry eye.

6. The ophthalmic composition according to any one of claims 1 to 5, wherein a content of 3-[(3S,4R)-3-methyl-6-(7H-pyrrolo[2,3-d]pyrimidin-4-yl)-1,6-diazaspir o[3.4]octan-1-yl]-3-oxopropanenitrile or a salt thereof is 0.01% by mass to 1% by mass based on a total amount of the ophthalmic composition.

7. The ophthalmic composition according to any one of claims 1 to 6, wherein the composition is an eye drop.
